# EUROPEAN PATENT APPLICATION

(11) **EP 1 522 596 A2**
(43) Date of publication of application: **13.04.2005**
(21) Application number: 04020470.3
(22) Date of filing: 27.08.2004
(51) Int. Cl.: C12Q 1/68, G01N 33/50

(54) **A method for the analysis of activation pathways controlled by neurotransmitters**

(30) Priority: 05.09.2003 US 655531
(71) Applicant: EPPENDORF AG, 22331 Hamburg (DE)
(72) Inventor: Remacle, José, 5020 Malonne (BE); de Longueville, Francois, 5360 Natoye (BE)
(74) Representative: Becker Kurig Straus

(57) **Abstract**

The invention pertains to a novel method for analyzing activation pathways controlled by neurotransmitters and to a micro-array for use in this method. In particular, the present method relates to the use of such a micro-array as a tool for investigation of several activation pathways in the rat brain, said activation pathways being under the control of amine receptors.

## Description

### FIELD OF THE INVENTION

The invention pertains to a novel method for analyzing activation pathways controlled by neurotransmitters and to a micro-array for use in this method. In particular, the present method relates to the use of such a micro-array as a tool for investigating several activation pathways in the brain of an animal, said activation pathways being under the control of amine receptors.

### BACKGROUND OF INVENTION

The brain of vertebrate animals comprises several parts, each of them being specialized and controlled by different neurotransmitters, which have individual receptors to which they bind.

A receptor is a protein associated with a cellular membrane and binding to one or more specific molecules only. Based on their signal transduction mechanisms they may be classified in four groups: (i) receptors that are ligand-gated ion channels (LGIC), e.g., the nicotinic acetylcholine and gamma-aminobutyric acid (GABA) receptors; (ii) receptors that are enzymes, e.g. the insulin receptor (a tyrosine kinase) or the atrial natriuretic peptide receptor, (a form of a guanylate cyclase); (iii) receptors, that couple to guanosine triphosphate (GTP)-binding proteins (GPCR receptors), e.g., muscarinic acetylcholine receptors; and (iv) receptors with unknown signal transduction mechanisms (e.g., the sigma receptor).

GPCR receptors transmit the signal of an externally bound signaling molecule across the cell membrane to activate heterotrimeric transducing proteins which bind GDP (guanosine diphosphate). Upon activation, the bound GDP is converted to GTP (guanosine triphosphate). The activated G protein complex then triggers further intracellular biochemical activities in the cell. Within the human genome, several hundred GPCRs have been identified so far and endogenous ligands are known for approximately 100 of this group.

From a classification point of view the GPCR receptors may be considered as a superfamily, with some members being part of the rhodopsin like, secretin like, metabolic glutamate/pheromone, fungal pheromone, cAMP receptor and the frizzled/smoothenened class of receptors.

The rhodopsin like class again comprises of different families including e.g. the peptide ligand-receptors, the aminergic-receptors, the hormone-receptors, the olfactory-, prostanoid-, nucleotide-like-, cannabinoid-, platelet activating factor-, gonadotropin-releasing hormone-, tyrotropin-releasing hormone-, melatonin-, viral-, lysosphingolipid-, leukotriene B4- and the orphan receptors. Each of the families is also divided in subfamilies. For example, the aminergic receptors subfamily comprises the dopamine (adrenergic), choline, histamine, serotonin and cholinergic, octopamine and trace amine receptors and each of the subfamilies are again divided into subtypes and sometime into sub-subtypes.

Much of the knowledge about the structure of GPCRs has been derived from the study of adrenergic receptors. The identification of two new dopamine receptors in the past decade beside three others already known, has led to a renewed enthusiasm for the study of the potential role of dopaminergic dysfunction in neuropsychiatric conditions, especially in psychotic disorders and substance abuse. The cloning of the D2 dopamine receptor in 1988 and the subsequent identification of multiple dopamine receptors referred to as D1, D3, D4, and D5 has changed the understanding of dopamine receptor anatomy and pharmacology.

The dopamine receptors are not equally distributed in the rat CNS. In general, the D 1 and D2 receptor mRNAs are more abundant in the CNS as compared to their pharmacologically related counterparts (Tiberi, M. et al, Neurobiology 88 (1991), 7491-7495,). D3 and D4 receptors appear to be localized primarily in limbic regions of rat brain, with little to no expression is found in the dorsal striatum. The D4 receptor seems to be of clinical importance because of its high affinity for atypical neuroleptics (O'Malley, K.et al, New Biol. **4**(2) (1992), 137-46). The D5 receptor has a very restricted distribution, primarily in the diencephalon and hippocampus (Khan, Z. et al, Neuroscience, **100**(4) (2000), 689-699).

Histamine (H-)receptors play an important role in the regulation of several (patho)physiological processes. In the mammalian brain, histamine itself (the neurotransmitter) is synthesized in a restricted population of neurons located in the tuberomammillary nucleus of the posterior hypothalamus. These neurons diffusely project to most cerebral areas and have been implicated in several brain functions (sleep/wakefulness, hormonal secretion, cardiovascular control, thermoregulation, food intake and memory). Four subtypes have been identified. H1 receptors are widely distributed in cerebral areas: cerebellum, hippocampus, thalamus, hypothalamic nuclei, nucleus accumbens, amygdaloid nuclei, and frontal cortex (Ryu, J. et al., Brain Res. Dev. **87**(2) (1995), 101-10). H2 receptors are found in most areas of the cerebral cortex, with the highest density in the superficial layers, the piriform and occipital cortices, both with low H1-receptor densities. H3 shows high concentration in striatum, nucleus accumbens, substantia nigra and certain cortex areas (Cumming, P. et al., Synapse **8**(2) (1991), 144-51). H4 receptors are expressed primarily in bone marrow (Liu, C. et al., Mol. Pharmacol. **59**(3) (2001), 420-6).

Acetylcholine (Ach) receptors as member of cholinergic receptors in the mammalian CNS may be divided into muscarinic (Chrm) and nicotinic (Chrn) subtypes based on the ability of the natural alkaloids, muscarine and nicotine, to mimic the effects of ACh as a neurotransmitter. The muscarinic receptors belong to the superfamily of GPCRs while nicotinic receptor are LGIC receptors.

In the brain, evidence suggests a role for muscarinic receptors in memory function and in the pathophysiology of affective illness and schizophrenia. Because of their putative role in cognitive function, muscarinic receptors have been a focus of research in Alzheimer's disease. Muscarinic receptors are widely distributed throughout the body. They mediate various types of responses throughout the peripheral and central nervous system (Department of Pharmacology, Pharmacol. Ther. 58(3) (1993), 319-79). Five subtypes have been identified. The M1 subtype is abundant in forebrain and sympathetic ganglia. The M2 subtype is expressed at a relatively low, uniform density throughout the brain. The M3 muscarinic receptor is expressed in relatively low density throughout the brain. The M4 muscarinic receptor is expressed abundantly in various regions of the forebrain, particularly the corpus striatum and olfactory tubercle. The M5 receptor represents less than 2% of the total density of muscarinic receptors in various regions of the brain (Ching-Fong Liao et al, The Journal of Biological Chemistry, **264**(13) (1989), 7328-7337).

Preclinical and clinical studies indicate that neuronal nicotinic receptor may have a substantial role in mediating antinociception, cognitive performance, modulating affect, and enhancing the release of other neurotransmitters. In rat, the nicotinic receptors are abundant in selective areas of the cerebral, thalamus, interpeduncular nucleus and the superior colliculus, but are of low to moderate abundance in the hippocampus and hypothalamus. Fourteen nicotinic receptor subtypes have been identified.

Preclinical studies investigating the physiological mechanisms of fear and anxiety states have strongly suggested that noradrenergic receptors are involved in the pathophysiology of human anxiety. The modem concept of alpha- and beta-adrenergic receptors (ARs) was introduced in 1948 by Ahlquist, who studied the effects of catecholamines on a variety of physiological responses. These included contraction and relaxation of the uterus, dilation of the pupil and stimulation of myocardial contraction. He demonstrated that norepinephrine (NE), epinephrine (Epi), isoproterenol (Iso), methylnorepinephrine, and methylepinephrine could cause either contraction or relaxation of smooth muscle, depending on the amine, its dose and the site of action. This pharmacological concept of distinct catecholamine receptors was further solidified with the development of highly selective antagonists. Six alpha and three beta receptor subtypes have been identified.

The alpha-1A-AR, alpha-1B-AR and alpha-1D-AR subtypes are expressed in the cerebral cortex. Analysis of various rat brain regions showed that thalamus and cerebral cortex had the highest proportion of the alpha1-B-AR.

The neurotransmitter serotonin (5-hydroxytryptamine, 5-HT) mediates a wide range of physiological functions by interacting with multiple receptors, and these receptors have been implicated as playing important roles in certain pathological and psychopathological conditions. In the CNS, serotonin is thought to be involved in learning and memory, sleep, thermoregulation, motor activity, pain, sexual and aggressive behaviors, appetite, neuroendocrine regulation, and biological rhythms. Serotonin has also been linked to pathophysiological conditions such as anxiety, depression, obsessive-compulsive disorders, schizophrenia, suicide, autism, migraine, emesis, alcoholism and neurodegenerative disorders.

Molecular cloning has indicated that 5-HT receptors belong to at least two protein superfamilies: G-protein-associated receptors which have seven putative transmembrane domains (TMDs) (5-HT1A/B/C/D/E, 5-HT2, 5-HT4, 5-HT5, 5-HT6, 5-HT7) and ligand gated ion channel receptors which have four putative TMDs (5-HT3). To date, 14 serotonin receptors have been identified in 7 subfamilies based on a structural homology, second messenger system activation, and drug affinity for certain ligands. The 5-HT2 subfamily is divided into 3 classes: 5-HT2A, 5-HT2B, and 5-HT2C. 5-HT2A and 5-HT2C receptor antagonists are thought to be useful in treating depression, anxiety, psychosis, and eating disorders. 5-HT2A and 5-HT2C receptors exhibit 51% amino acid homology overall and approximately 80% homology in the transmembrane domains. The 5-HT2C receptor was cloned in 1987 and led to the cloning of the 5-HT2A receptor in 1990. Studies of the 5 HT2A receptor in recombinant mammalian cell lines revealed that the receptor possessed two affinity states, high and low. Both the 5-HT2A and 5-HT2C receptors are coupled to phospholipase C and mediate responses through the phosphatidylinositol pathway (Saudou, F. et al, Neurochemistry 25(6) (1994), 503-32). Studies with agonists and antagonists display a wide range of receptor responses suggesting that there is a wide diversity of regulatory mechanisms governing receptor activity. The 5-HT2A and 5-HT2C receptors have also been implicated as the site of action of hallucinogenic drugs.

In addition to the classical neurotransmitter-amines, there also exists a class of trace amines that are found in very low levels in mammalian tissues and include tyramine, B-phenylethylamine (B-PEA), tryptamine and octopamine. Although there is literature that supports a role for trace amines in depression as well as other psychiatric disorders and migraine, the role of trace amines as neurotransmitters in mammalian systems has not been thoroughly examined. Since they share common structures with the classical amines and may displace other amines from their storage vesicles, trace amines have been referred to as "false transmitters".

Thus, many of the effects of trace amines are indirect and are caused by the release of endogenous classical amines. Nevertheless, levels of these amines are altered in various disorders. At present, 14 subtypes have been described in the rat brain, the two first members of this family (TA1 and TA2) being well characterized. TA1 is activated most potently by tyramine and B-PEA, and displays a low affinity for tryptamine and octopamine. TA2 is also activated by B-PEA and tryptamine. Although the roles of tyramine and octopamine as neurotransmitters acting via stimulation of GPCR in invertebrate systems are well established, mammalian GPCRs for trace amines have not been reported so far.

The control of these receptors for brain function is effected through activation pathways, wherein each of the subtypes of the subfamily of amine being responsible for the pathway activation and for a certain response of the part of the brain to the stimulus or to the inhibition of the activation.

There have been several attempts to develop tools for the detection of GPCR receptors.

US-2003013137 discloses a method for detecting GPCR activity which comprises providing at least one cell expressing a GPCR and a plurality of conjugated proteins. Each of the conjugated proteins is formed by linking an arrestin protein to a detectable molecule. The conjugated proteins are contacted with the cell and are substantially evenly distributed in the cytoplasm thereof. A first image of the cell is obtained by detecting an amount of energy emitted from the detectable molecules. Also, the cell is treated with an agonist whereupon a second image of the cell is obtained. The first image and the second image are compared to detect the localization of at least some of the of conjugated proteins in/at endocytic vesicles and/or endosomes.

WO 02/052047 describes a method for isolating novel GPCRs whose expression is associated with disease states. The invention is based on the elongation of oligonucleotide probes immobilized on a solid support. Each probe group corresponds to a particular region within the reference sequence of a GPCR, and contains at least four sets of probes with the first set being exactly complementary to the particular region of the reference sequence, and the other three sets being identical to the first set on their 3'-end sequence. In practicing the invention, a reaction mixture comprising the appropriate target polynucleotides mixed with the reagents necessary for conducting the polymerase chain reaction (PCR) are placed in contact with each immobilized primer pair or single primer population on the solid support. The reaction mixture can also contain a label molecule capable of being incorporated into the nascent strands during polymerase chain reaction so that the amplified products can be detected on the solid support after the PCR.

WO 02/095065 discloses a micro-array for the determination of the expression of GPCR polynucleotides belonging to the peptide family, wherein at least 2 polynucleotide sequences are present on the arrays to differentiate the receptors from the other different peptide receptors.

Since a number of neurotransmitter receptors has been associated with profound changes in mental activity and functioning, it is assumed that abnormal activity of these receptors may contribute to certain psychiatric disorders. Therefore, the potential and real therapeutic utility of receptor agonists and antagonists over a number of diseases has led to the creation of plurality of targeted drugs for central nervous system (CNS) disorders.

In view of their central role in these activities and as -potential targets for future drugs there is a need in the art to obtain specific and precise detailed information about the neurotransmitter receptors related to the ligand families.

### Definitions

In the context of the present application and invention the following definitions apply :

The term "expressed genes" are the parts of the genomic DNA which are transcribed into mRNA and then translated into a peptides or proteins. The measurement of the expressed genes is performed on either molecules within this process most currently the detection of the mRNA or of the peptide or protein. The detection can also be based on specific property of the protein being for example its enzymatic activity.

The terms "nucleic acid, array, probe, target nucleic acid, bind substantially, hybridizing specifically to, background, quantifying" are as described in the international patent application WO97/27317, which is incorporated herein by way of reference.

The term "nucleotide triphosphate" refers to nucleotides present in either as DNA or RNA and thus includes nucleotides which incorporate adenine, cytosine, guanine, thymine and uracil as bases, the sugar moieties being deoxyribose or ribose. Other modified bases capable of base pairing with one of the conventional bases adenine, cytosine, guanine, thymine and uracil may be employed. Such modified bases include for example 8-azaguanine and hypoxanthine.

The term "nucleotide" as used herein refers to nucleosides present in nucleic acids (either DNA or RNA) compared with the bases of said nucleic acid, and includes nucleotides comprising usual or modified bases as above described.

References to nucleotide(s), polynucleotide(s) and the like include analogous species wherein the sugar-phosphate backbone is modified and/or replaced, provided that its hybridization properties are not destroyed. By way of example the backbone may be replaced by an equivalent synthetic peptide, called Peptide Nucleic Acid (PNA).

The terms "nucleotide species" is a composition of related nucleotides for the detection of a given sequence by base pairing hybridization; nucleotide species are from the same family of closely related sequences which will hybridized with one specific target sequence, the non specific hybridization being not significant or as low so as not to interfere with the quantification of the specific target. Typically capture probe species are one oligonucleotide sequence synthesized chemically and having side products arising from the fact that the synthesis is not obtained at 100% so that some low amount of related sequences are present within the main sequence. Also, polynucleotides sequences synthesized by enzymatic amplification may contain some mis-incorporation of nucleotides but in general at such a low level that it does not interfere with the capture probe's task to serve as a specific capture of the target. The essential characteristic of a nucleotides species for the invention is that the overall species can be used for capture of a given sequence belonging to a given gene.

The term "polynucleotide" sequences that are complementary to one or more of the genes described herein, refers to polynucleotides that are capable of hybridizing under stringent conditions to at least part of the nucleotide sequence of said genes. Polynucleotides also include oligonucleotides being below 100 bases which can be used under particular conditions. Such hybridizable polynucleotides will typically exhibit at least about 75% sequence identity at the nucleotide level to said genes, preferably about 80% or 85% sequence identity or more preferably about 90% or 95% or more nucleotide sequence identity to said genes. They are composed of either small sequences typically 15-30 base long or longer ones being between 30 and 100 or even longer between 100 and 800 base long.

The term "bind(s) substantially" refers to complementary hybridization between a probe nucleic acid and a target nucleic acid and embraces minor mismatches that can be accommodated by reducing the stringency of the hybridization media to achieve the desired detection of the target polynucleotide sequence.

The term "capture probe" designates a molecule which is able to specifically bind to a given polynucleotide or polypeptide. Polynucleotides binding is achieved via base pairing between two polynucleotides, one being the immobilized capture probe and the other one the target to be detected. Polypeptide binding is best performed using antibodies specific for the polypeptide for the capture of a given polypeptide or protein. Part of the antibodies, or recombinant proteins incorporating part of the antibodies, typically the variable domains, or even proteins being able to specifically recognized the peptide can also be used as capture probes.

The terms "background" or "background signal intensity" refers to hybridization signals resulting from non-specific binding, or other interactions, between the labeled target nucleic acids and components of the polynucleotide array (e. g. the polynucleotide probes, control probes, the array substrate, etc.). Background signals may also be produced by intrinsic fluorescence of the array components themselves. A single background signal can be calculated for the entire array, or a different background signal may be calculated for each target nucleic acid. In a preferred embodiment, the background is calculated individually for each spot, being the level intensity of the signal around each of the spot.

The phrase "hybridizing specifically to" refers to the binding, duplexing or hybridizing of a molecule substantially to or only to a particular nucleotide sequence or sequences under stringent conditions when that sequence is present in a complex mixture (e. g., total cellular) DNA or RNA.

The "hybridized nucleic acids" are typically detected by detecting one or more "labels" attached to the sample nucleic acids. The labels may be incorporated by any of a number of means well known to those of skill in the art, such as detailed in WO 99/32660, which is incorporated herein by way of reference.

The term "capture probes" in the sense of the present invention shall designate genes or parts of genes of different length, e.g. between 10 and 1500 nucleotides, which are either synthesized chemically in situ on the surface of the support or laid down thereon. Moreover, this term shall also designate polypeptides or fragments thereof, or antibodies directed to particular polypeptides, which terms are used interchangeably, attached or adsorbed on the support.

The term "homology" is intended to mean the degree of identity of one polynucleotide to another polynucleotide. According to the invention the term homology is used in connection with complementarity between polynucleotides within a family. There may be complete homology (i. e. 100% identity) between two or more polynucleotides. The degree of homology may be determined by any method well known for a person skilled in the art.

The term "ligand-gated ion channels" (LGIC) are proteins inserted into the plasma membrane of cells especially in the neuronal cells and which upon activation or inactivation in the presence of ligands open or close the channel for influx of ions. LGIC include the nicotinic acetylcholine and gamma-aminobutyric acid (GABA) receptors.

The term "GPCR" is intended to mean a polypeptide which is a transmembrane protein consisting of seven transmembrane domains and which interact with 3 G coupled proteins (Ga, Gb, Gg) for transferring an activation or inactivation signal due to an agonist or antagonist present on the surface of the receptor. The majority of the GPCR's have the ability to transduce a signal across the cell membrane through activation of G-proteins (bound to GTP) on the intracellular side of the cell. However, some of these receptors may also be signaling via alternative signal molecules like Jak2 kinases, phospholipase Cy or protein kinase C. The GPCR superfamily also include the classes of Rhodopsin, secretin like.

The term "GPCR or LGIC subtypes" are the GPCR or LGIC from the same family which are related to the same neurotransmitters as ligands but which differ in their sequence. Subtypes of the GPCR and LGIC for 5 neurotransmitters of 3 animal species are presented in table 1.

The term "GPCR or LGIC polynucleotide" is intended to mean a polynucleotide encoding a polypeptide (GPCR or LGIC) involved in transducing a signal across biological membranes.

The term "GPCR or LGIC polynucleotide family" is intended to mean polynucleotides encoding polypeptides of a GPCR or LGIC family". The polynucleotides actually sequenced may be found and downloaded from Genbank or EMBL (see e. g. http: www. ncbi. nih. org). GPCR's and LGIC's are direct or indirect targets for the action of many compounds, such as drugs acting on activation or inactivation of cells. Because of their involvement into the neuronal activity drugs acting on the receptors cover a large spectrum of pharmacological activity.

The term "biological material" includes within its meaning organisms, organs, tissues, cells or biological material produced by a cell culture. The biological material may be living or dead. The material may correspond to one or more cells from the organisms, in case the organism is a multicellular organism, the material may correspond to one or more cells from one or more tissues creating the multicellular organism. The biological material to be used according to the invention may be derived from particular organs or tissues of the multicellular organism, or from isolated cells obtained from a single or multicellular organism. In obtaining the sample of RNA's to be analyzed from the biological material from which it is derived, the biological material may be subject to a number of different processing steps. Such steps might include tissue homogenization, cell isolation and cytoplasma extraction, nucleic acid extraction and the like and such processing steps are generally well known for a person skilled in the art. Methods of isolating RNA from cells, tissues, organs or whole organisms are known to those skilled in the art and are described in e. g. Sambrook et al., Molecular Cloning : A Laboratory Manual, Cold Spring Harbor Press (1989). The biological material may be of the same kind i. e. the biological material is of the same kind of origin, such as coming from the same type of tissue, the same organism or the same type of organism or the same cell type etc.

The term "tissue" is intended to mean a collection of differentiated cells such as adrenal gland, total brain, liver, heart, kidney, lung, pancreas, mammary gland, placenta, prostate, salivary gland, skeletal muscle, small intestine, spleen, stomach, testis, thymus, trachea, and uterus.

The term "brain tissue" includes, but is not restricted to, the cortex, striatum, hippocampus, cerebellum, olfactory bulb, limbic regions, hypothalamus, thalamus, nucleus accumbens, amygdaloid nuclei and substantia nigra

The term "target polynucleotide" is intended to mean a polynucleotide present in the biological material of interest. The target polynucleotide encodes a polypeptide, which is at least a part of a amine receptor. If the target polynucleotide has a complementary polynucleotide present on the array, it will hybridize thereto and thus give rise to a detectable signal.

### Brief description of the drawings and tables

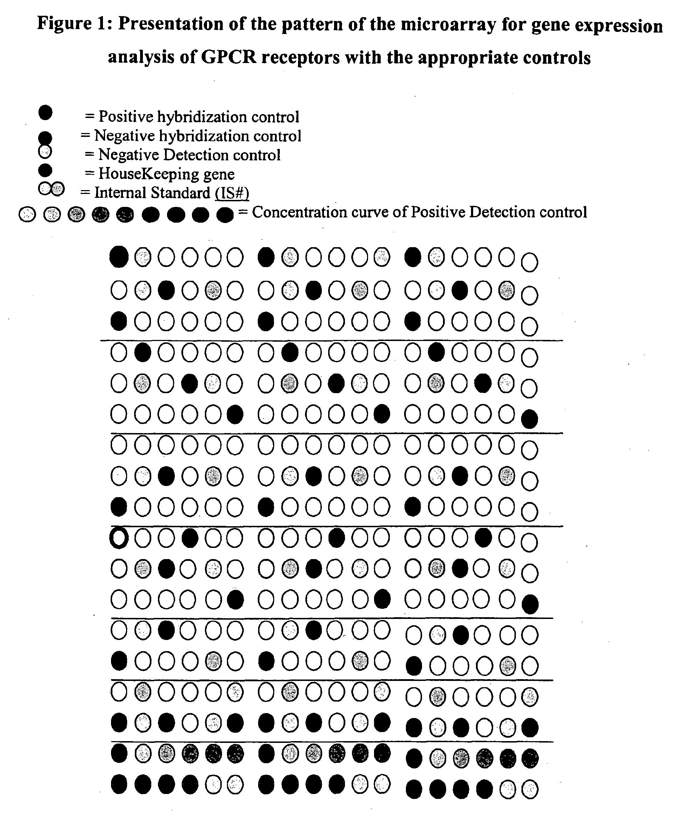

Fig. 1 shows the micro-array giving an overview of the gene expression of the 46 different subtypes for 5 neurotransmitter receptors in different parts of a tissue by single analysis of the data present on the array pattern.

Table 1 lists Amine ligand GPCR and LGIC receptor subtypes issued from http://www.gpcr.org.

Table 2 provides the description of activation pathways which are under the control of receptor subtypes and some data on the heterogeneous distribution in different parts of the brain.

Table 3 provides results obtained according to the method described in invention for the determination of differentially expressed amine receptor genes in different regions of the brain (example 1 : the cortex versus the striatum) and in different tissues (example 2 : the liver versus the brain).

### DETAILED DESCRIPTION OF THE INVENTION

The invention provides a method for simultaneously analyzing the status of different activation pathways present in the different parts of the brain of animals, which different pathways are under the control of 5 major subfamilies of the amine neurotransmitters. The method comprises the step of obtaining nucleic acids from a biological sample and contacting the nucleic acids with a micro-array, containing on specific locations thereon at least one capture probe derived from a gene encoding a receptor for a dopamine, a histamine, a serotonine, an adrenergic and a cholinergic neurotransmitter, and determining the expression profile of said receptors in the biological sample, by evaluating the two dimensional pattern of data present as intensities of spots on the surface of the micro-array, one spot being sufficient for obtaining the information on one neurotransmitter subtype.

In a preferred embodiment the micro-array contains capture probes representing genes encoding -receptors of at least 3 for dopamine, at least 4 for histamine, at least 7 for serotonine, at least 3 for adrenergic and at least 7 for cholinergic neurotransmitters.

The invention also provides a tool for obtaining a general outline of the expression pattern of different subtypes of the 5 major subfamilies of neurotransmitter receptors of the GPCR and LGIC superfamilies including at least one capture probe derived from a gene encoding a receptor for a dopamine, a histamine, a serotonine, an adrenergic and a cholinergic neurotransmitter and in a preferred embodiment capture probes for at least 3 different subtypes for dopamine, 3 for histamine, 6 for serotonin, 3 for adrenergic and 7 for cholinergic receptors, more preferably including at least 20 different subtypes or sub-subtypes among 5 different subtypes for dopamine, 4 for histamine, 14 for serotonin, 5 for adrenergic and 16 for cholinergic.

According to an alternative preferred embodiment the tool also comprises at least one of the 14 subtypes of trace amine receptors.

The present method and tool allows the specific determination of the expression status in one assay of at least 60% of the various subtypes and sub-subtype genes of the GPCR or LGIC receptors regulated by all amine neurotransmitters, as exemplified by the list provided in table 1. This represents for the rat, mouse and human receptors, 5 different subtypes for dopamine, 4 for histamine, 14 for serotonin, 5 for adrenergic and 16 for cholinergic and 14 for the trace amine beside some possible subtypes being commonly detected on the same capture probe.

The gist of the present invention resides in providing data about a certain number of neurotransmitter receptors present in the different parts of the brain and being responsible for different types of cell activation through different pathways, which data give a general overview of the expression of the different receptors, and thus the status of the brain tissue under investigation. Normally the data about the different subtypes of the receptors obtained from different parts of the brain or from the same part under a different physiological/chemical condition are compared. The ratios of the presence of the different subtypes are calculated and related to the biological effect which is to be investigated. In general, a high expression of the subtypes are linked to an activation of the pathways for which they are responsible and which are taken to explain the respective biological effect.

Examples for different subtypes of receptors to be detected and analyzed in different parts of the brain in a single assay are presented in tables 1 and 2 and a preferred array/composition of the tool according to the present invention is shown in figure 1. Table 2 also provides the description of the activation pathways which are under control of the receptor subtypes and some data on the heterogeneous distribution in the different parts of the brain.

Due to the publication of the human genome, essentially all of the sequences for human receptors are known and publicly available, e.g. from public databases, such as the National Center for Biotechnology Information (NCBI) or the LocusLink web site (http://www.ncbi.nlm.nih.gov/LocusLink/). Here, information are provided about an official nomenclature, aliases, sequence accessions, phenotypes, EC numbers, MIM numbers, UniGene clusters, homology, map locations, and related web sites. For other mammary animals many GPCR and LGIC sequences are known and information stored in the web site http://www.gpcr.org/. From these sequences, a skilled person may design appropriate capture probes for the specific detection of the gene nucleotide sequences.

The capture probes for the amine receptors are of the GPCR type or of the LGIC types. The GPCR type receptors for the amine ligands are presented in table 1 and their role in the different activation pathways in the different parts of the brain in vertebrates is presented in table 2. The LGIC for the amine are mainly the nicotinic acetylcholine receptors, the most important being CHRNA2,3,4,5,7 and B1,2,3,4 and the D and E subtypes. The serotonin receptors are the Htr3A and Htr3B types. These receptor subtypes are preferentially provided together with the GPCR receptors for getting a full overview of the activation pathways occurring in the different parts of the brain (Table 2). Exemplarily named other genes, that may serve as clinical indicators in striatum, are preproenkephalin 2, Tachykinin , GPR 88 and in cortex are Thioredoxin, Poly(c)-binding protein 4.

An array according to the invention is exemplarily presented in figure 1. Beside the GPCR and LGIC receptors associated with the amine as ligands, the micro-arrays may also contain some genes of interest for the analysis of a pathological condition associated with the brain, such as Parkinson or epilepsy. Also, controls required for a quantification of the genes are present. These include negative and positive controls, internal standards and house keeping genes. The results obtained with such a tool for the determination of the differentially expressed amine receptor genes in different parts of the brain is presented in table 3. The results were obtained on rat tissue since the capture probes present on the array were specific of the rat genes. Similar array can be constructed for other mammalian species including mouse and human with some adaptation in the sequence for the capture probes.

According to an embodiment of the invention, the variations of the gene expression fulfill the following criteria in order to be relevant for interpretation :
1) These variations are recognized as being, or at least suspected to be, of biological relevance for understanding the mechanism of cell activation but they are also of diagnostic, prognostic, or predictive value.
2) They are expressed in cells preferentially in neuronal cells at levels detectable by the DNA chip. The present invention gives also a means of providing micro-array with a direct detection of 46 subtypes of 5 amine neurotransmitter receptors (figure 1) having the genes with cDNA prepared from as low as 1 to 10 µg of total RNA.
3) Variations of mRNA amount is parallel to variations of the corresponding protein and gene expression. The amount of mRNA at a given moment in the cells reflects the equilibrium between transcription and degradation. This amount is also influenced by gene deletions and amplifications. Protein structures and activities are the ultimate support of cell functions and tumor properties. A good correlation between mRNA and protein levels may be crucial when it concerns these therapeutic target proteins, The polynucleotide detection is favorably replaces by the polypeptide detection in order to directly obtained the quantification of the transcript of the genes.

In the preferred embodiment, the tool for the analysis of the overall subtypes of receptors is a micro-array solid support on which are present capture molecules being single stranded DNA attached on specific locations, each location being covered by one species of capture probe for the detection and quantification of one subtype or closely related subtypes.

In the present invention, one spot is sufficient for identifying one gene. The direct relationship between the spot identification and the subtype identification makes the invention particularly useful as a tool for screening the subtypes in different samples since the overview of the results on the array directly corresponds to the overview of the subtypes expression in the sample or in the tissue.

In principle, the micro-array may contain as few as 20 capture probes, i.e. one capture probe associated with each of 3 different subtypes for dopamine, 3 for histamine, 5 for serotonin, 3 for adrenergic and 6 for cholinergic. Yet, the number of capture probes on the micro-array may be selected according to the need of the skilled person and may contain capture probes for the detection of up to about 3000 different genes, e.g. about 100, or 200 or 500 or 1000, or 2000 different genes being either other subtypes of other receptors or genes for proteins involved in the cell functions or even house keeping genes for quantification purpose.

According to a preferred embodiment capture probes are long polynucleotides and are unique for each of the genes to be detected and quantified on the array. Long capture probes mean capture probes of from about 15 to about 1000 nucleotides in length, preferably of from about 15 to about 400, or 15 to 200 nucleotides, or more preferred of from about 15 to about 100, and are fixed on a support being any solid support as long as they are able to hybridize with their corresponding cDNA and be identified and quantified. The density of the capture nucleotide sequences bound to the surface of the solid support is preferably above to 3 fmoles per cm² of solid support surface.

The nucleic acid to be detected is either RNA from a specific brain tissue of interest or cDNA obtained therefrom, the latter of which is easier to handle. The cDNA may be obtained by retrotranscription from total RNA or mRNA. To this end, total RNA is extracted from tissue in an amount of about 0.1 to 100 µg, preferably about 0.1. to 50 µg, more preferably about 0.1. to 20 µg, even more preferably about 0.1 to 10 µg, or even more preferred between about 0.1 and 2 µg and is directly used for hybridization on the array. mRNA may also be processed in the same way with a much lower amount to be used for the copying into cDNA.

When RNA is amplified by means of a T7 polymerase based method, PCR, rolling circle or other methods, detection is possible even at a concentration lower than 0.1 µg of total RNA or mRNA depending on the amplification obtained being usually in the order of a few hundreds for the T7 polymerase and much higher for the PCR or rolling circle amplifications. In extreme cases, the investigation of a single cell or a group of a few cells, such as obtained by laser dissection methods is feasible. In amplification methods, however, different genes are amplified with different efficiencies and corrections may have to be provided.

According to another preferred embodiment the original nucleotide sequences to be detected and/or to be quantified are RNA sequences are copied by the retro-transcription of the 3' or 5' end wherein consensus primer and possibly also a stopper sequence are used. Preferably, the copied or amplified sequences are detected without previous cutting of original sequences into smaller portions.

When preparing cDNA, the DNA may at the same time be labeled, e.g. by including in the enzymatic process nucleotides harboring an appropriate label. Alternatively, a label may be attached to the nucleic acid, including, for example, nick translation or end-labeling by attachment of a nucleic acid linker joining the sample nucleic acid to a label. In an alternative embodiment, also the capture probes attached to the support may be labeled.

Detectable labels suitable for use in the present invention include any composition detectable by spectroscopic, photochemical, biochemical, immunochemical, electrical, optical or chemical means. Useful labels in the present invention include biotin for staining with labeled streptavidin conjugate, magnetic beads (e.g., Dynabeads™), fluorescent dyes (e.g., cyanine dyes, such as Cy5, fluorescein, texas red, rhodamine, green fluorescent protein, and the like), radiolabels (e.g., ³H, ¹²⁵I, ³⁵S, ¹⁴C, or ³²P), enzymes (e.g., horse radish peroxidase, alkaline phosphatase and others commonly used in an ELISA), and colorimetric labels such as colloidal gold or colored glass or plastic (e.g., polystyrene, polypropylene, latex, etc.) beads. Patents teaching the use of such labels include U.S. Patent Nos. 3,817,837; 3,850,752; 3,939,350; 3,996,345; 4,277,437; 4,275,149; and 4,366,241.

Also, variants of the genes may specifically be detected and quantified on the array. In some tissues, multiple mRNAs are transcribed from the same gene. These variants often exhibit more or less overlapping sequences. Selection of the capture probes has to be specific for such marker, when all variants do not have the same potential importance as diagnostic, prognostic, or predictive indicator, or when it is recommended to detect only some specific ones.

Receptors sub-subtypes are sometimes closely related so that the corresponding capture probes may be designed to be capable to differentiate between these closely related sub-subtypes or to recognize all of the sub-subtypes. For example a capture probe may be designed common for all subtypes Adra 2a, 2b, 2c and giving an overall analysis of the Adra 2 subtype receptors, while capture probes, distinguishing between the sub-subtypes may be designed, which are e.g. shown in table 3.

The target nucleic acid is contacted with the micro-array under conditions allowing hybridization of complementary strands only. Hybridization processes and conditions are well known in the art and are described e.g. in Sambrook et al., Molecular Cloning: A Laboratory Manual, 2nd Edition, Cold Spring Harbor Laboratory Press, Cold Spring Harbor, N.Y. (1989).

E.g. the hybridization step may be performed under a cover glass in hybridization chambers or in a hybridization oven which essentially represent a diffusion limited process. Alternatively, the reaction may be carried out with agitation, which decrease the required hybridization time. When using hybridization involving movement of the micro-array, the hybridization time may be reduced to a period of 60 minutes or less, preferably to 30 minutes or less. As a result thereof, the total assay can be performed in less than 4, more preferably 3.5 hours.

After hybridization with the target nucleotides of the biological sample the spot intensity is read according to the label utilized, e.g. in fluorescence or colorimetry. The quantification of the genes may be performed by standard techniques, e.g. by comparison with internal standards introduced in the retro-transcription of mRNA.

In case the target nucleic acid has been labeled prior to contacting it with the micro-array, the signal and/or the signal intensity is a means for determining the activation of a particular pathway or a change thereof. Alternatively, in case the capture probes are provided labeled, in this case the hybrids formed are cleaved by an appropriate enzyme, e.g. RNAse H in case of using the RNA pool for hybridization, while liberating the label. Hence in this embodiment the loss of a signal is indicative of an activation of a particular pathway of a change thereof.

The support as such may be made from any material conventionally used for this purpose and is preferably selected from the group consisting of glasses, electronic devices, silicon supports, silica, metal or mixtures thereof prepared in format selected from the group of slides, discs, gel layers and/or beads.

The present invention also pertains to a diagnostic and/or prognostic kit, which comprises means and media for performing the above method.

Specifically, a gene expression analysis may be performed on the present micro-array by contacting the nucleic acid derived from cells or tissues that had been subject to a particular or different environmental situation, e.g. had been exposed to a physical challenge or had been incubated in the presence of (potential) drugs and/or are derived from a different type of neuronal tissue. Then the data about the expression of the genes investigated are compared with the expression profile either from cells or tissues that had not been subjected to a particular or different environmental situation or from another cell population optionally comprising neuronal cells or not.

Based on the array identification of gene expression, the effect of the drugs or chemical may then be evaluated for its potential activity. Therefore, the present invention also comprises the use of the present micro-array in the diagnostic, prognostic and treatment of neuronal diseases. E.g. a Parkinson disease is associated with the overexpression of subtypes Drd1a and Drd2 of dopamine receptors.

In one of the embodiment, the invention provides a method of screening for an agent capable of modulating the onset or progression of a disease associated with neuronal performance, such as Parkinson, epilepsy, Alzheimer, depression, anxiety, ageing, comprising the steps of exposing a cell to the agent and detecting the expression level of at least one gene associated with each of at least 4 of the 5 neuronal receptors subtypes.

According to the present invention, potential drugs may be screened to determine, if the application of the drug alters the expression of the genes referred to herein. This may be useful, for example, in determining whether a particular drug is effective in treating a particular patient with neuronal diseases, especially degenerative diseases such as Alzheimer, Parkinson, epilepsy, depression. In the case where a gene expression is affected by the potential drug such that its level of expression returns to normal, the drug is indicated in the treatment of this disease. Similarly, a drug which causes expression of a gene which is not normally expressed by neuronal or glials cells in the brain, may be contraindicated in the treatment of the disease.

One of the main embodiment is the study of chemical compound having an agonist or antagonist effect on the receptors and to link the amount level of the subtype with the physiologically observed effect. The correlation between the expression of one or several subtypes of receptors allows to investigate the use or the development of the compound or one compound from the same family as a drug to specifically affect this or these subtypes. In this way the invention is particularly useful in the testing of agonist or antagonist of the neurotransmitters as acting specifically or preferentially on one of the subtype.

Assays to monitor the expression of a marker or markers, as e.g. defined in table 1 may utilize any available means of monitoring for changes in the expression level of the nucleic acids of the invention. As used herein, an agent is said to modulate the expression of a nucleic acid of the invention, if it is capable of up-or down-regulating expression of the nucleic acid in a cell.

Agents that are assayed for in the above methods may be selected randomly or rationally or may be also designed. As used herein, an agent is said to be randomly selected when the agent is chosen randomly without considering the specific sequences involved in the association of the protein of the invention alone or with its associated substrates, binding partners, etc. An example of randomly selected agents is the use a chemical library or a peptide combinatorial library, or a growth broth of an organism.

The genes for the 5 neurotransmitter receptors identified as being differentially expressed in neuronal cells may be used in a variety of nucleic acid detection assays to detect or quantify the expression level of a gene or multiple genes in a given sample. For example, traditional Northern blotting, nuclease protection, RT-PCR and differential display methods may be used for detecting gene expression levels.

The protein products of the genes identified herein can also be assayed to determine the amount of expression. Methods for assaying for a protein include Western blot, immunoprecipitation, radioimmunoassay and protein chips. Protein chips are supports bearing as capture probes antibodies or related proteins specific of the different proteins or peptides to be analyzed. Antibodies are either on the same support as a protein array or are on different supports as beads, each beads being specific for the detection of proteins. It is preferred, however, that the mRNA be assayed as an indication of expression.

Any hybridization assay format may be used, including solution-based and solid support-based assay formats. A preferred solid support is a low density array also known as a DNA chip or a gene chip. In one assay format, the array containing probes to at least 3 gene associated with each of the 5 neurotransmitters subtypes as e.g. listed in table 1 may be used to directly monitor or detect changes in gene expression in the treated or exposed cell as described herein. Assays of the invention may determine the expression level of about 14, 50, 100, 400, 1000 or even 3000 genes with some or all derived from table 2.

According to the present invention the number of genes to be detected is limited since this allows to better focus on genes useful for the characterization of neuronal cells and to give a more rapid and precise response to the questions of the prognostic, diagnostic and therapeutic follow up of the patients. The larger the number of genes to be analyzed and treated for data mining, the less efficient the correlation and the outcome of the analysis.

In another assay format, cells or cell lines are first identified, which express one or more of the gene products mentioned herein physiologically. Cells and/or cell lines thus identified will comprise the necessary cellular machinery to ensure that the transcriptional and/or translational apparatus mimics the response of normal or pathological brain tissue to an exogenous biological or physical change, such as the presence of a chemical agent. Such machinery would likely include appropriate surface transduction mechanisms and/or cytosolic factors. The tool as provided by this invention is then applied on these cells or cell lines and the invention is used as a research tool for investigating the role of the different amine neurotransmitter receptors in the tested experimental condition.

In another embodiment, the invention further envisages computer systems comprising a database containing information identifying the expression level in neuronal cell or brain tissue of at least one gene associated with each of the 5 neuronal receptor subtypes as listed in table 1 and a user interface to view the information. The database may further include sequence information for the genes, information identifying the expression level for the set of genes in normal neuronal tissue and may contain links to external databases, such as GeneBank. The present invention includes relational databases containing sequence information, for instance for one or more of the genes of table 1, as well as gene expression information in various breast tissue samples. Databases may also contain information associated with a given sequence or tissue sample such as descriptive information about the gene associated with the sequence information, descriptive information concerning the clinical status of the tissue sample, or information concerning the patient from which the sample was derived. The database may be designed to include different parts, for instance a sequence database and a gene expression database. Methods for the configuration and construction of such databases are widely available, for instance from US-5,953,727, which document is incorporated herein by reference by way of reference.

The following examples illustrate the invention without limiting it thereto.

### Example 1:

The striatum is considered as the control and the cortex as the test.

The data are presented in the table 3 as ratio of test versus control.

### 1. RNA extraction:

Poly (A)+ RNA was isolated from rat frozen tissues using the FastTrack 2.0 mRNA isolation Kit (Invitrogen) according to manufactures protocol. To assess the integrity and relative contamination of mRNA with ribosomal RNA, analysis on bioanalyser (Agilent) were carried out.

The concentration and purity of RNA was determined by diluting an aliquot of the preparation in TE (10mM Tris-HCl pH 8, 1mM EDTA) and measuring (reading) its absorbance (in a spectrophotometer) at 260 nM and 280 nm. While A260 allows to evaluate the RNA concentration, the A260/A280 ratio gives an indication of RNA purity. For a RNA to be used, its ratio must be comprised between 1.8 and 2.

### 2. cDNA synthesis:

1 µl of poly(A+) RNA sample (0.5 µg/µl) was mixed with 2 µl oligo(dT)12-18 (0.5 µg/µl, Roche), 3.5 µl H2O, and 2 µl of a solution of 6 different synthetic well-defined poly(A+) RNAs. These latter served as internal standards to assist in quantification and estimation of experimental variation introduced during the subsequent steps of analysis. After an incubation of 10 minutes at 70°C and 5 minutes on ice, 9 µl of reaction mix were added. Reaction mix consisted in 4 µl Reverse Transcription Buffer 5X (Gibco BRL), 1 µl RNAsin Ribonuclease Inhibitor (40 U/ml, Promega), and 2 µl of a 10X dNTP mix, made of dATP, dTTP, dGTP (5 mM each, Roche), dCTP (800 µM, Roche), and Biotin-11-dCTP (800 µM, NEN).

After 5 minutes at room temperature, 1.5 µl Superscript II (200 U/ml, Gibco BRL) was added and incubation was performed at 42°C for 90 minutes. Addition of Superscript and incubation were repeated once. The mixture was then placed at 70°C for 15 minutes and 1 µl Ribonuclease H (2U/µl) was added for 20 minutes at 37°C. Finally, a 3-minutes denaturation step was performed at 95°C. The biotinylated cDNA, was kept at -20°C.

### 3. Hybridization with biotinylated cDNA:

The array used in this study is composed of 60 genes and 8 housekeeping genes and is presented in figure 1. Several different controls including positive and negative detection control, positive and negative hybridization control, six different internal standards are all dispersed at different locations among the genes to be analyzed on the micro-array. In this example each spots was covered with a capture probe being a polynucleotide species which allow the specific binding of one target polynucleotide corresponding to a specific gene listed in table 2.

Hybridization chambers were from Biozym (Landgraaf, The Netherlands). Hybridization mixture consisted in biotinylated cDNA (the total amount of labeled cDNA), 6.5 µl HybriBuffer A (Eppendorf, Hambourg, Germany), 26 µl HybriBuffer B (, Eppendorf, Hambourg, Germany), 8 µl H2O, and 2 µl of positive hybridization control.

Hybridization was carried out overnight at 60°C. The micro-arrays were then washed 4 times for 2 min with washing buffer (B 1 0.1X + Tween 0.1%) (Eppendorf, Hamburg, Germany).

The micro-arrays were than incubated for 45 minutes at room temperature with the Cy3-conjugated IgG Anti biotin (Jackson Immuno Research laboratories, Inc #200-162-096) diluted 1/1000 X Conjugate-Cy3 in the blocking reagent and protect from light.

The micro-arrays were washed again 5 times for 2 minutes with washing buffer (B 1 0.1X + Tween 0.1%) and 2 times for 2 minutes with distilled water before being dried under a flux of N2.

### 4. Scanning and data analysis:

The hybridized micro-arrays were scanned using a laser confocal scanner "ScanArray" (Packard, USA) at a resolution of 10 µm. To maximize the dynamic range of the assay the same arrays were scanned at different photomultiplier tube (PMT) settings. After image acquisition, the scanned 16-bit images were imported to the software, 'ImaGene4.0' (BioDiscovery, Los Angeles, CA, USA), which was used to quantify the signal intensities. Data mining and determination of significantly expressed gene in the test compared to the reference arrays was performed according to the method described by Delongueville et al (Biochem Pharmacol. 2002 Jul 1;64(1):137-49). Briefly, the spots intensities were first corrected for the local background and than the ration between the test and the reference arrays were calculated. To account variation in the different experimental steps, the data obtained from different hybridizations were normalized in two ways. First the values are corrected using a factor calculated from the intensity ratios of the internal standard reference and the test sample. The presence of 3 internal standard probes at different locations on the micro-array allows measurement of a local background and evaluation of the micro-array homogeneity, which is going to be considered in the normalization (Schuchhardt et al., Nucleic Acids Res. 28 (2000), E47). However, the internal standard control does not account for the quality of the mRNA samples, therefore a second step of normalization was performed based on the expression levels of housekeeping genes. This process involves calculating the average intensity for a set of housekeeping genes, the expression of which is not expected to vary significantly. The variance of the normalized set of housekeeping genes is used to generate an estimate of expected variance, leading to a predicted confidence interval for testing the significance of the ratios obtained (Chen et al, J. Biomed. Optics 1997, 2:364-74). Ratios outside the 95% confidence interval were determined to be significantly changed by the treatment.

The results of the experiment are presented in table 3. The results are presented as the ratios between the genes expressed in the two samples. The results are the values for the genes which significantly changed in the two samples. The striatum is considered as the control and the cortex as the test. We can easily observed the variation in the expression level of the different genes associated with subtypes of the receptors in the two parts of the brain which were analyzed in this experiment mainly the cortex and the striatum.

### Example 2:

### Gene expression in different tissues

Comparison of receptors subunits expression in the brain and the liver.

The experiment was performed as described in the example 1 with the proviso of using whole brain and liver as tissues. The 4 steps were RNA extraction, cDNA synthesis, hybridization on the array and the quantification and analysis of the results.

The results of the experiment are presented in table 3. The results are presented as the ratios between the genes expressed in the two samples. The results are the values for the genes which significantly changed in the two samples. The brain is considered as the control and the liver as the test. The variation in the expression level of the different genes associated with subtypes of the receptors in the two tissues which were analyzed in this experiment could be easily monitored.

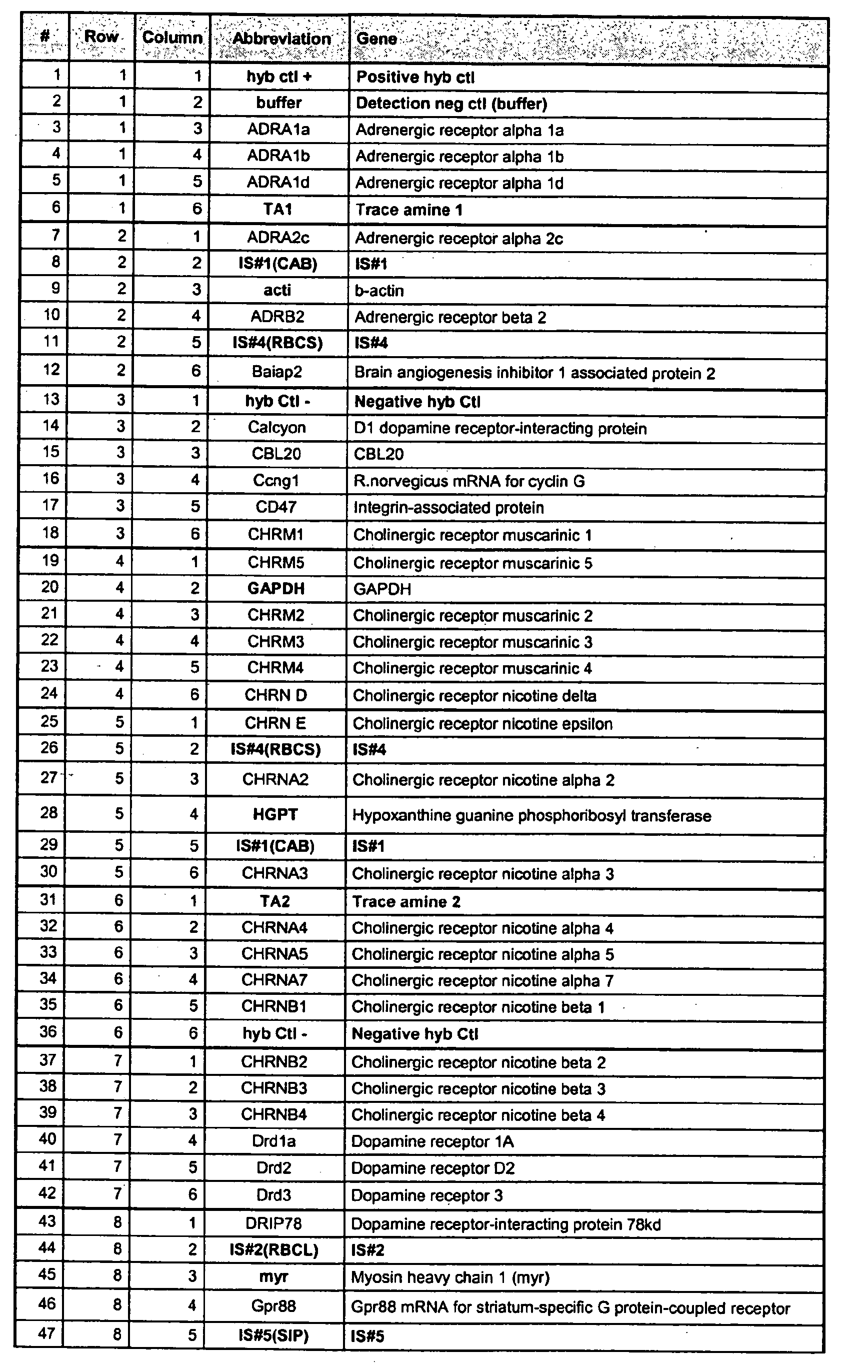

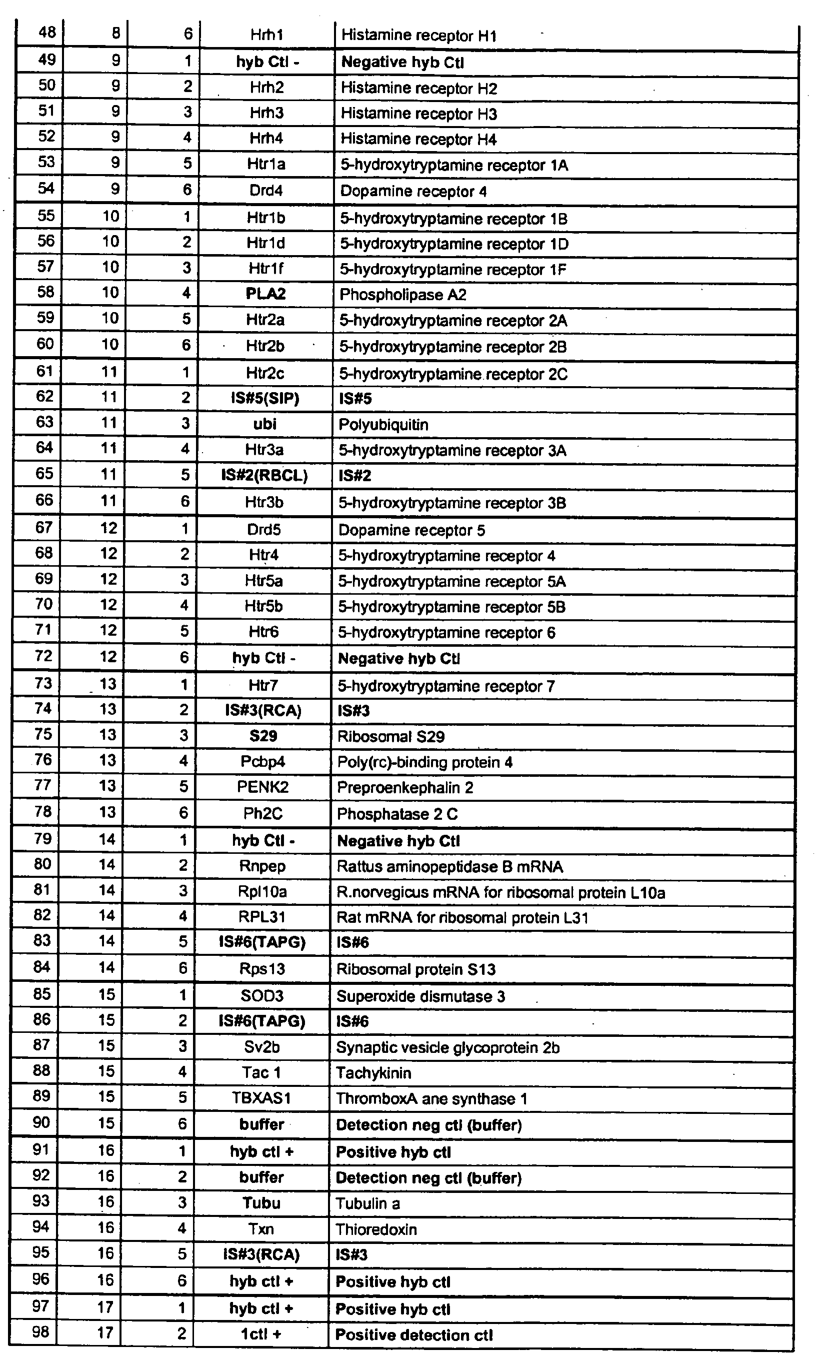

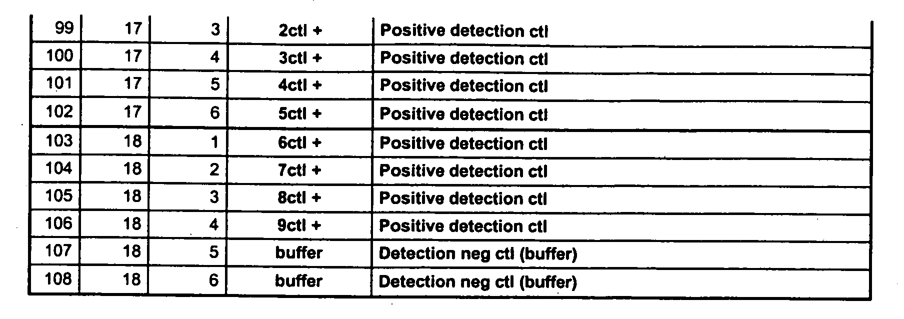

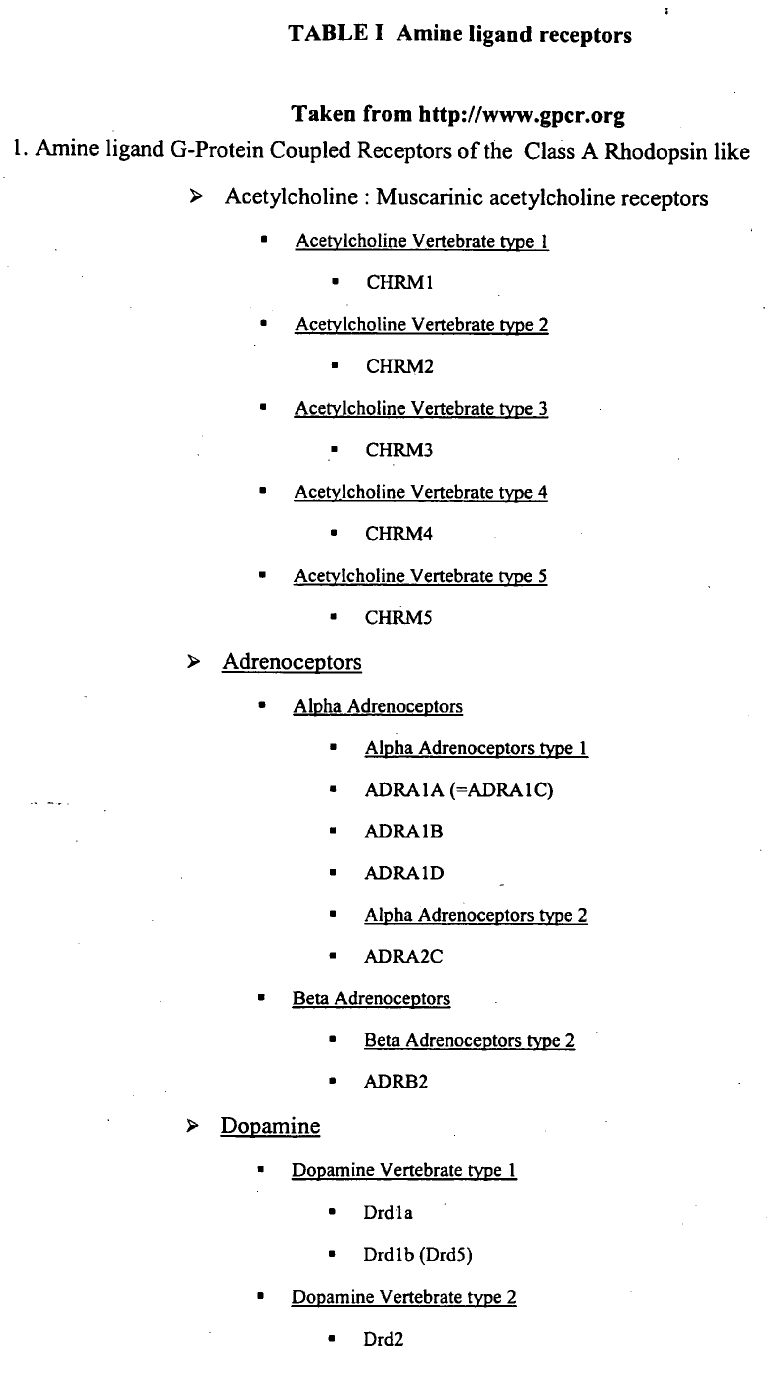

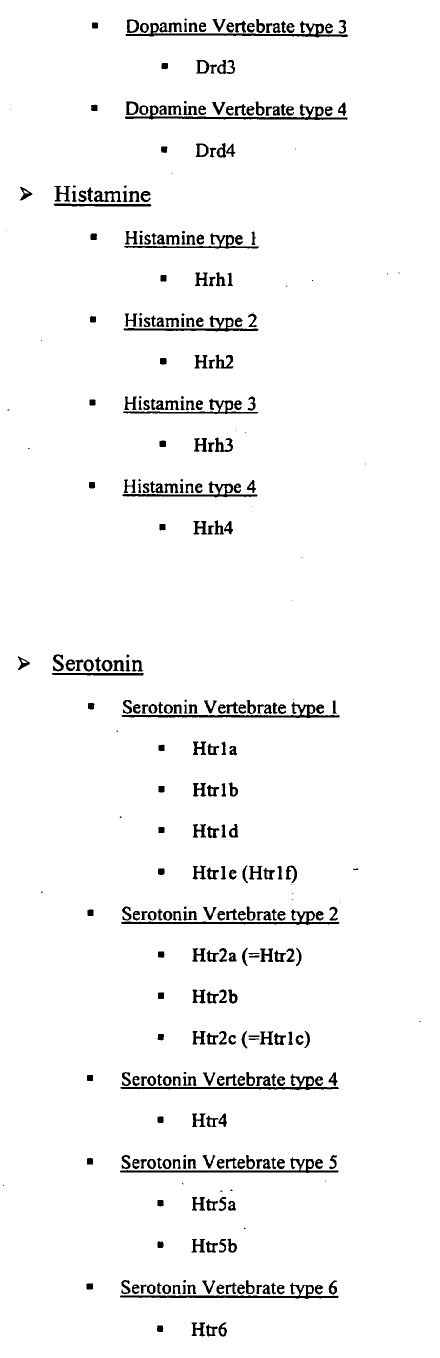

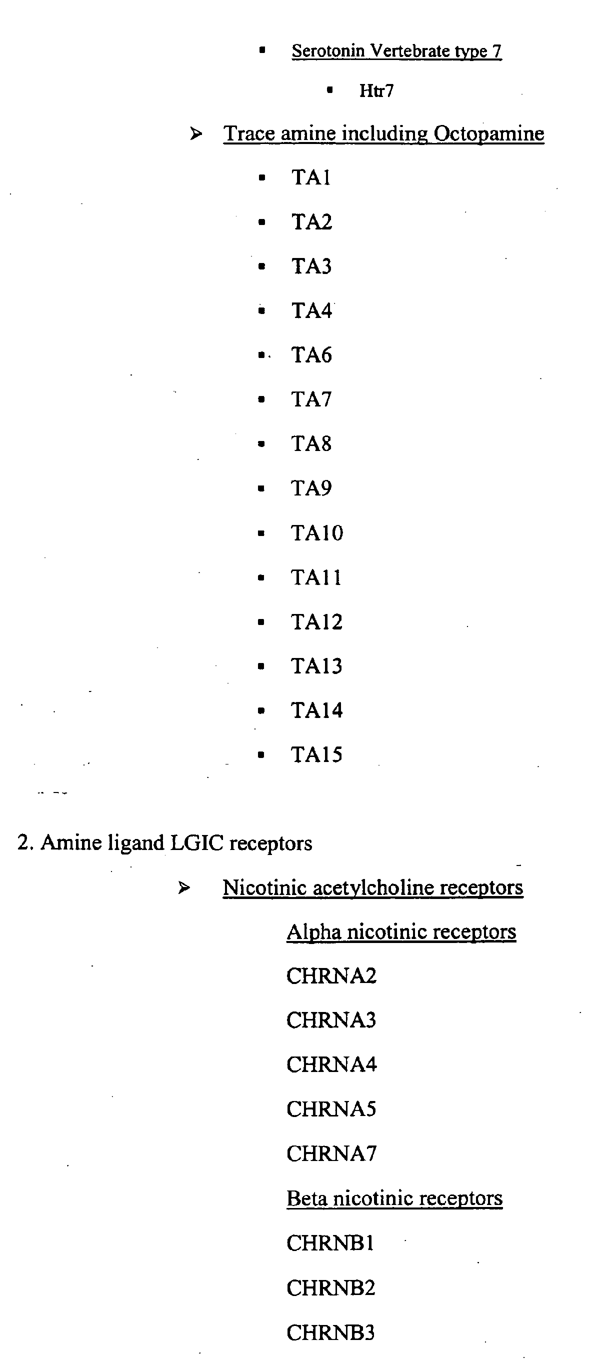

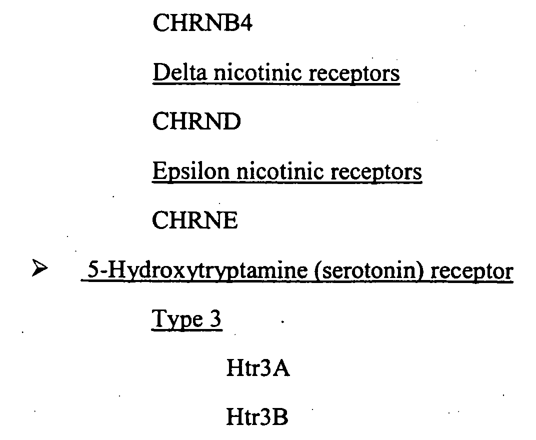

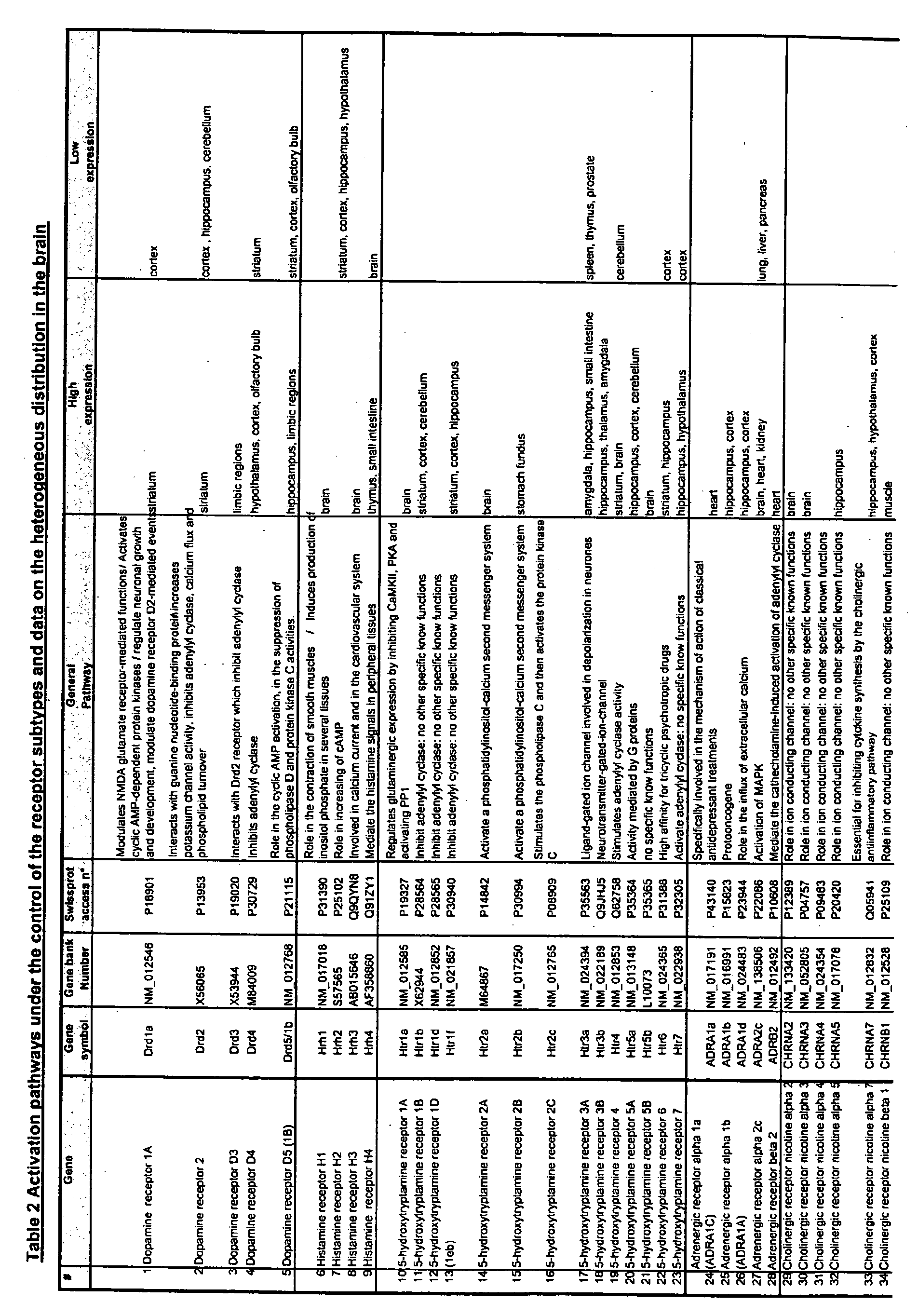

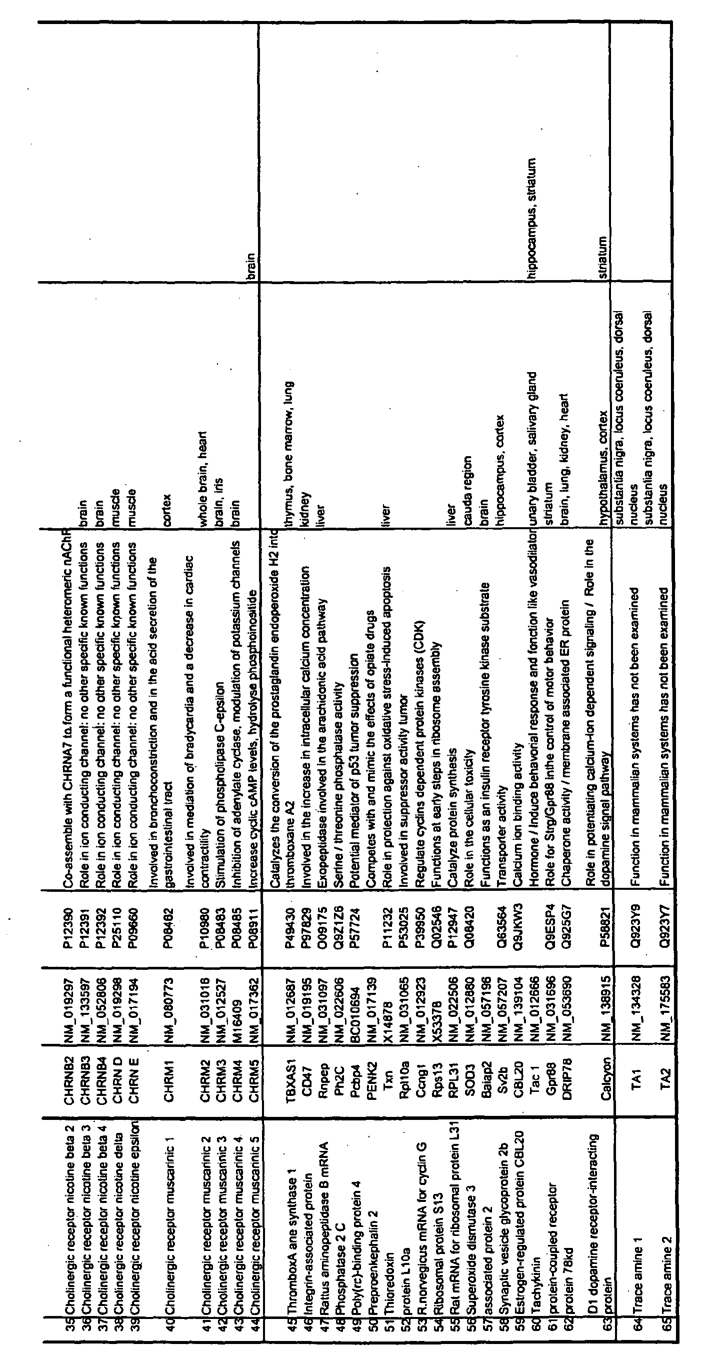

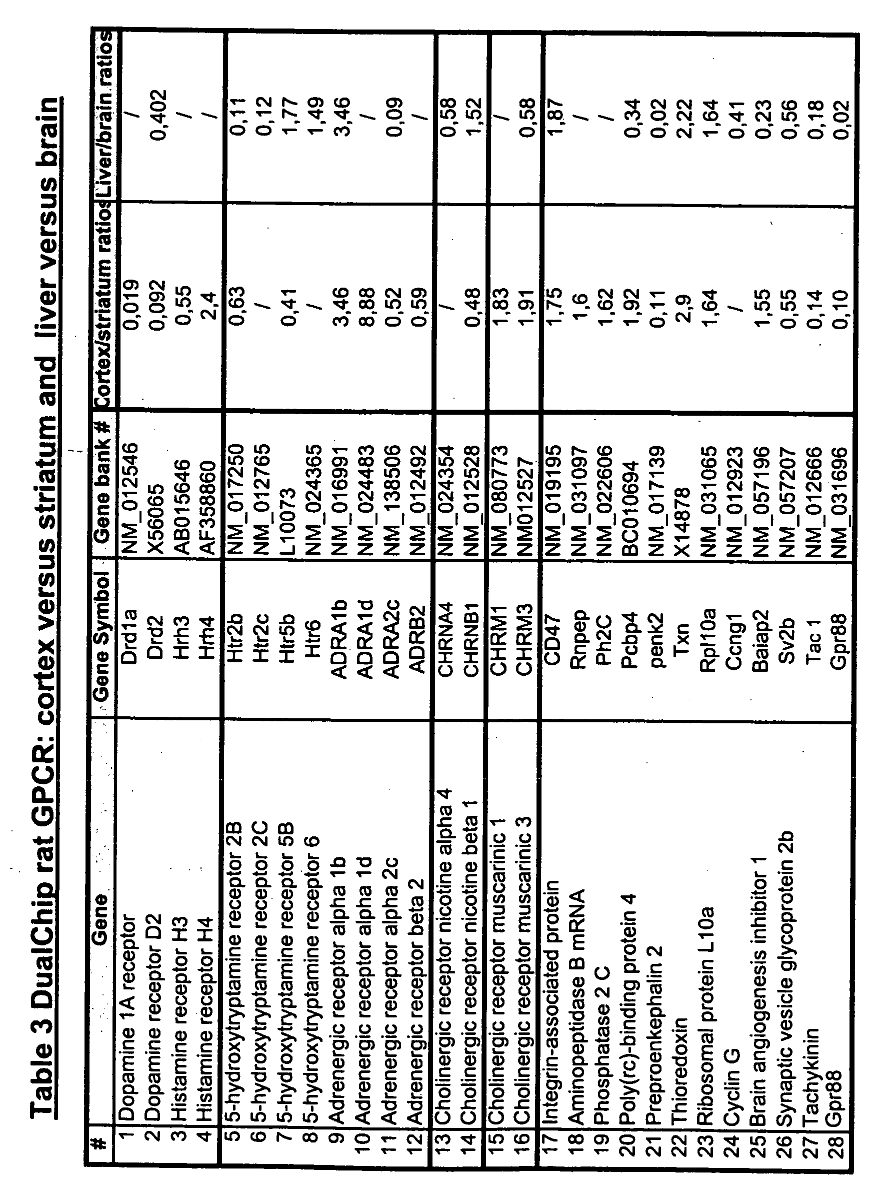

## Claims

1. A method for analyzing activation pathways controlled by neurotransmitters comprising,
(i) obtaining a nucleic acid from a biological sample;
(ii) contacting the nucleic acid with a micro-array comprising capture probes derived from the 5 major subfamilies of amine neurotransmitter receptors, under conditions allowing hybridization of complementary strands; and
(iii) analyzing a two dimensional pattern of data present as intensities of spots on the surface of a support of the micro-array, one spot being sufficient for obtaining the information on one neurotransmitter subtype.

2. A method for evaluating the activity of a chemical compound on brain tissue comprising,
(i) contacting brain tissue or a cell culture derived therefrom with a chemical compound;
(ii) isolating nucleic acid from cells of the brain tissue or culture;
(iii) optionally amplifying the nucleic acid obtained;
(iv) contacting the nucleic acid with a micro-array comprising capture probes derived from the 5 major subfamilies of amine neurotransmitter receptors, under conditions allowing hybridization of complementary strands; and
(v) analyzing the two dimensional pattern of data present as intensities of spots on the surface of a support of the micro-array, and comparing the data obtained with data obtained with a tissue or cells that had not been in contact with the chemical compound; and
(vi) comparing the data obtained from the different samples.

3. The method according to claims 1 and 2, wherein the micro-array comprises capture probes specific for at least 2 subtypes of dopamine receptors, 2 subtypes of histamine receptors, 4 subtypes of serotonin receptors, 2 subtypes of adrenergic receptors and 4 subtypes of cholinergic receptors.

4. The method according to claims 1 and 2, wherein the micro-array comprises capture probes for at least 20 different subtypes or sub-subtypes among the 5 subtypes for dopamine, 4 subtypes for histamine, 14 subtypes for serotonin, 5 subtypes for adrenergic and 16 subtypes.

5. The method according to claim 4, wherein the micro-array includes capture probes for the detection of 1 subtype of octopamine and the 14 subtypes of trace amines.

6. The method according to claims 1 and 2, wherein the capture probes are derived from the list given in table 1.

7. The method according to claims 1 and 2, wherein the capture probes are derived from the sense or from the antisense strand of the gene encoding the receptor.

8. The method according to claims 1 and 2, wherein the target nucleic acid derived from a biological sample is selected from RNA or cDNA.

9. The method according to claims 1 and 2, wherein the nucleic acid is labeled during synthesis of cDNA.

10. The method according to claim 9, wherein the label comprises fluorescent dyes, radiolabels, enzymes or colorimetric labels.

11. The method according to claim 2, wherein the tissue to be investigated is the cortex, striatum, hippocampus, cerebellum, olfactory bulb, limbic regions, hypothalamus, thalamus, nucleus accumbens, amygdaloid nuclei, substantia nigra or an extract obtained from any of these.

12. A method for identifying a compound useful for treating a neurological disorder, comprising,
(i) contacting brain tissue or a cell culture derived therefrom with a chemical compound;
(ii) isolating nucleic acid from cells of the brain tissue or culture;
(iii) optionally amplifying the nucleic acid obtained;
(iv) contacting the nucleic acid with a micro-array comprising capture probes derived from the 5 major subfamilies of amine neurotransmitter receptors, under conditions allowing hybridization of complementary strands; and
(v) analyzing the two dimensional pattern of data present as intensities of spots on the surface of a support of the micro-array, and comparing the data obtained with data obtained with a tissue or cells that had not been in contact with the chemical compound; and
(vi) comparing the data obtained from the different samples.

13. The method according to claim 12, wherein the compound is capable of modulating the onset or progression of a disease associated with neuronal performance, such as Parkinson, epilepsy, Alzheimer, depression, anxiety or aging.

14. The method according to claim 13, wherein the compound is capable of modulating the expression level of at least one gene associated with each of at least 4 of the 5 neuronal receptor subtypes.

15. A micro-array for analyzing activation pathways controlled by neurotransmitters, comprising capture probes derived from the 5 major subfamilies of amine neurotransmitter receptors.

16. The micro-array according to claim 15,
further comprising capture probes specific for at least 2 subtypes of dopamine receptors, 2 subtypes of histamine receptors, 4 subtypes of serotonin receptors, 2 subtypes of adrenergic receptors and 4 subtypes of cholinergic receptors; or
further comprising capture probes for at least 20 different subtypes or sub-subtypes among the 5 subtypes for dopamine, 4 subtypes for histamine, 14 subtypes for serotonin, 5 subtypes for adrenergic and 16 subtypes for cholinergic; or
wherein the micro-array includes capture probes for the detection of 1 subtype of octopamine and the 14 subtypes of trace amines; or
wherein the capture probes are derived from the list given in table 1; or
wherein the capture probes are derived from the sense or from the antisense strand of the gene encoding the receptor; or
further comprising capture probes fixed onto 2 or more solid supports.

17. A diagnostic kit comprising a micro-array comprising capture probes derived from the 5 major subfamilies of amine neurotransmitter receptors.
